# EUROPEAN PATENT APPLICATION

(11) **EP 1 050 757 A1**
(43) Date of publication of application: **08.11.2000**
(21) Application number: 99108722.2
(22) Date of filing: 03.05.1999
(51) Int. Cl.: G01N 33/68, A61K 38/18

(54) **Methods of diagnosing or treating Alzheimer's disease on basis of increased cerebrospinal fluid levels of nerve growth factor**

(71) Applicant: Evotec BioSystems AG, 22525 Hamburg (DE)
(72) Inventor: The designation of the inventor has not yet been filed
(74) Representative: Meyers, Hans-Wilhelm, Dr.Dipl.-Chem.

(57) **Abstract**

The invention relates to a method for diagnosing or prognosing Alzheimer's disease in a subject, or determining whether a subject is at increased risk of developing Alzheimer's disease, comprising:
determining a level of nerve growth factor and/or of a transcription product of a gene coding for nerve growth factor in a sample from said subject;
and comparing said level to a reference value representing a known disease or health status,
thereby diagnosing or prognosing said Alzheimer's disease in said subject, or determining whether said subject is at increased risk of developing Alzheimer's disease.

## Description

Alzheimer's disease (AD), first described by the Bavarian psychiatrist Alois Alzheimer in 1907, is a progressive neuropsychiatric disorder which begins with short term memory loss and proceeds to loss of cognitive functions, disorientation, impairment of judgement and reasoning and, ultimately, dementia. It is the most common form of dementia. The neuropathology is characterized by the formation in brain of amyloid plaques and neurofibrillary tangles. AD has been estimated to afflict 5 to 11 percent of the population over age 65 and as much as 47 percent of the population over age 85. Moreover, as adults, born during the population boom of the 1940's and 1950's, approach the age when AD becomes more prevalent, the control and treatment of AD will become an even more significant health care problem. Familial forms of AD are genetically heterogeneous, but most with early onset are linked to mutations in the presenilin genes *PSEN1* and *PSEN2,* as well as to mutations of the amyloid precursor gene *APP.* The majority of AD patients have no obvious family history and are classified as sporadic AD. For this late onset AD, several putative genetic risk factors have been reported. Among these the ApoE-epsilon 4 (ApoE 4) has been widely confirmed to confer increased risk for AD. Inheritance of ApoE4 and other risk factors are neither necessary nor sufficient to cause AD. In contrast to the APP- and PSEN mutations which increase the production of Aβ, the principal component of senile plaques in AD brain, the ApoE variant most likely influences Aβ accumulation by modulating clearance and degradation of the peptide.

In the search for biochemical changes in patients with neuropsychiatric disorders analysis of cerebrospinal fluid (CSF) may be a useful method, since the CSF is continuous with the extracellular fluid of the brain. Therefore, a plurality of studies aiming at the analysis of the central nervous system (CNS) specific proteins in CSF were performed in order to find biochemical markers for neuronal and synaptic function and pathology in degenerative brain disorders.

Nerve growth factor (NGF) is one of the neurotrophic agents that promote differentiation or support the survival and functioning of some populations of neurons, influencing their effects not only on the peripheral sensory and sympathetic neurons but also on the central neurons. The pathophysiological roles of NGF in the human nervous system, especially in relation to neuropsychiatric disorders, has not been fully understood yet. It is known that patients with acute multiple sclerosis (MS), traumatic brain injury or hypertensive cerebral hemorrhage show higher NGF levels in the CSF and NGF has trophic roles in regenerating axons in the CNS.

To determine the pathophysiological roles of NGF in the human CNS with special reference to neuropsychiatric disorders, levels of NGF in CSF from patients with the following neurodegenerative disorders have been examined by Nisho et al. (Clinica Chimica Acta 275, 93 - 98, 1998) using a highly sensitive two-site enzyme immunoassay:
(i) Parkinson's disease
(ii) Progressive supranuclear palsy
(iii) Sporadic olivo-ponto-cerebellar atrophy
(iv) Spinocerebellar ataxia 3 / Machado-Joseph disease
(v) Dentato-rubro-pallido-luysian atrophy
However, Nisho et al. did not examine any patients suffering from Alzheimer's disease.

As AD is a growing social and medical problem, there is a strong need for methods of diagnosing or prognosing said disease in subjects as well as for methods of treatment.

In one aspect, the invention features a method for diagnosing or prognosing Alzheimer's disease in a subject, or determining whether a subject is at increased risk of developing Alzheimer's disease, comprising:
determining a level of nerve growth factor and/or of a transcription product of a gene coding for nerve growth factor in a sample from said subject;
and comparing said level to a reference value representing a known disease or health status,
thereby diagnosing or prognosing said Alzheimer's disease in said subject, or determining whether said subject is at increased risk of developing Alzheimer's disease.

It is preferred to use a body fluid, preferably cerebrospinal fluid, as said sample. An increase of a level of nerve growth factor in cerebrospinal fluid from a subject relative to said reference value representing a known health status indicates a diagnosis, or prognosis, or increased risk of said Alzheimer's disease in said subject.

In a further aspect, the invention features a method of monitoring progression of Alzheimer's disease in a subject, comprising:
determining a level of nerve growth factor and/or of a transcription product of a gene coding for nerve growth factor in a sample from said subject;
and comparing said level to a reference value representing a known disease or health status, thereby monitoring progression of said Alzheimer's disease in said subject.

It is particularly preferred to further compare a level of nerve growth factor and/or of a transcription product of a gene coding for nerve growth factor in said sample with a level of at least one of said substances in a series of samples taken from said subject over a period of time.

In still a further aspect, the invention features a method of evaluating a treatment for Alzheimer's disease, comprising:
determining a level of nerve growth factor and/or of a transcription product of a gene coding for nerve growth factor in a sample from said subject;
and comparing said level to a reference value representing a known disease or health status,
thereby evaluating said treatment for said Alzheimer's disease.

In another aspect, the invention features a kit for diagnosis, prognosis, or determination of increased risk of developing Alzheimer's disease in a subject, said kit comprising:
(a) at least one reagent which selectively detects nerve growth factor or a transcription product of a gene coding for nerve growth factor; and
(b) instructions for diagnosing, or prognosing Alzheimer's disease, or determining increased risk of developing Alzheimer's disease by
   (i) detecting a level of nerve growth factor and/or of a transcription product of a gene coding for nerve growth factor in a sample from said subject; and
   (ii) diagnosing, or prognosing, or determining whether said subject is at increased risk of developing Alzheimer's disease,
wherein an increased level of nerve growth factor and/or of a transcription product of a gene coding for nerve growth factor compared to a reference value representing a known health status;
or a level of nerve growth factor and/or of a transcription product of a gene coding for nerve growth factor similar or equal to a reference value representing a known disease status
indicates a diagnosis, or prognosis, or increased risk of developing Alzheimer's disease.

In another aspect, the invention features a method of treating or preventing Alzheimer's disease in a subject comprising administering to said subject in a therapeutically effective amount an agent or agents which directly or indirectly affect an activity, or level, or both said activity and level, of at least one substance which is selected from the group consisting of a gene coding for nerve growth factor, its non-coding regulatory elements, a transcription product of a gene coding for nerve growth factor, and nerve growth factor.

In still another aspect, the invention features the use of an agent for treating Alzheimer's disease, wherein said agent directly or indirectly affects an activity, or level, or both said activity and level, of at least one substance which is selected from the group consisting of a gene coding for nerve growth factor, its non-coding regulatory elements, a transcription product of a gene coding for nerve growth factor, and nerve growth factor.

The invention further features a method for identifying an agent that directly or indirectly affects an activity, or a level, or both said activity and level, of at least one substance which is selected from the group consisting of a gene coding for nerve growth factor, its non-coding regulatory elements, a transcription product of a gene coding for nerve growth factor, and nerve growth factor, comprising the steps of:
(a) providing a sample containing at least one substance which is selected from the group consisting of a gene coding for nerve growth factor, its non-coding regulatory elements, a transcription product of a gene coding for nerve growth factor, and nerve growth factor;
(b) contacting said sample with at least one agent;
(c) comparing an activity, or a level, or both said activity and level, of at least one of said substances before and after said contacting.

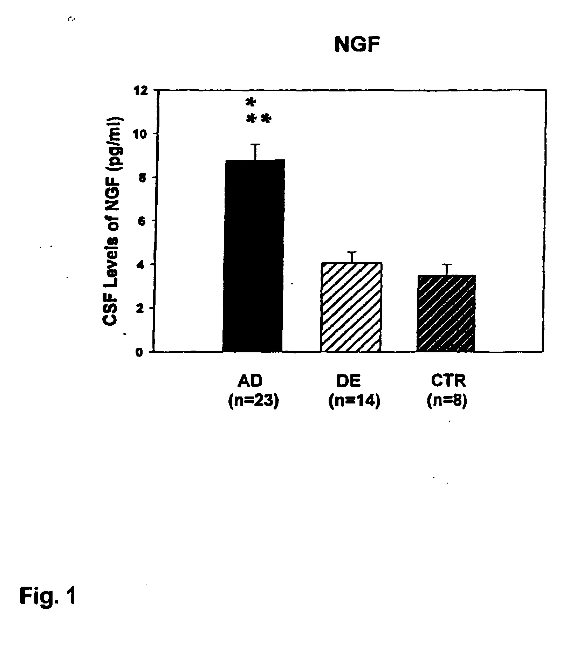

Figure 1 depicts that CSF levels of NGF are significantly elevated in the AD group, as compared to both the group consisting of patients with major depression (DE) as well as to the control group (CTR). Levels (pg/ml) are given in mean ± SEM. Asterisk (*, **) indicate significance (p< 0.05), Mann-Whitney U Test. * AD versus DE, p < 0.001; ** AD versus CTR, p < 0.001. NGF concentrations in CSF of the AD group amounted to 8.79 +/- 0.72 pg/ml (mean +/- SEM, range: 3.29 to 14.95, n = 23), compared to 4.07 +/- 0.50 pg/ml in the DE group (range: 2.42 to 9.54, n = 14), and 3.49 +/- 0.51 pg/ml in the CTR group (range: 0.00 to 4.64, n = 8), respectively. The alterations in patients suffering from AD may reflect disturbances in the trophic support of specific neuronal populations, such as the basal forebrain cholinergic system. There was no apparent correlation of CSF levels of NGF with ApoE genotype (or phenotype, respectively), age, duration of AD, MMS, NOSGER or MADRS scores.

### EXAMPLE 1

In order to achieve a differential diagnosis, the study included not only patients with AD, but also such with major depression (DE). Diagnosis of probable AD was made according to criteria of the National Institute of Neuropsychiatric and Communicative Disorders and Stroke-Alzheimer's disease and Related Disorders Association (NINCDS-ADRDA; McKhann et al., Neurology 34, 939 - 944, 1984). Patients with major depression were diagnosed according to the ICD10 (F32.0x/1x, F33.0x/1x) and DSM-IIIR (296.20-22, 296.30-32) criteria. All patients were referred to the research ward from general practitioners, neurologists and psychiatrists for diagnostic purposes and screening for clinical trials. None of the patients was institutionalized. The group of healthy control subjects (CTR) consisted of patients that underwent lumbar puncture for orthopedic or neurologic diagnostic purposes and were shown to have normal CSF cell counts, total protein levels, and absence of signs of blood barrier dysfunction or cerebral IgG synthesis, as well as absence of any cerebral disorders.

AD, DE and CTR patients were carefully examined and received a thorough clinical work-up. Psychometric testing including the Mini Mental State (MMS; Folstein et al., J. Psychiatry Res. 12, 189 - 198, 1975), as a global screening instrument for dementia, and the Nurses' Observation Scale for Geriatric Patients (NOSGER; Spiegel et al., J. Am. Geriatr. Soc. 39(4), 339 - 347, 1991) as a functional measure of dementia severity. The patients with DE showed no cognitive disturbances in the clinical examinations and the Mini Mental State scores were within the normal range. Severity of depression was rated by using the Montgomery Asberg Depression Rating Scale (MADRS) (Montgomery et al., Br. J. Psychiatry, 134: 382 - 389, 1979). Apolipoprotein (ApoE) genotyping, or, if DNA was not available, ApoE phenotyping was included in the laboratory screening in the AD patients.

CSF was obtained for diagnostic purposes in the AD and DE patients in which no lumbar puncture had been previously done during the routine diagnostic work-up. Different CSF volumes were available for the analysis of the neurotrophin proteins. This fact explains the different sample sizes for the individual measurements. All available CSF samples were used for the analyses.

The AD group was as follows: n = 23, 12 men, 11 women, mean age 63.9 +/- 13.2 SD years, range 39 - 86 years, MMS score: mean 18.6 +/- 5.6 SD.

The DE group was as follows: n = 14, 5 men, 9 women, mean age 68.2 +/- 13.6 SD years, range 47 - 86 years, MMS score: mean 28.1 +/- 0.9 SD.

The CTR group was as follows: n = 8, 5 men, 3 women, mean age 60.1 +/- 18.1 SD years, range 31 - 81 years.

AD and CTR patients were free of psychotropic medication. Patients with major depression were treated with various antidepressant drugs including serotonin reuptake inhibitors, reversible monoaminooxidase A inhibitors and tricyclics. Informed consent was taken from each patient and their caregivers before the investigation. The study was approved by the local ethics committee. All procedures were in accordance with the Helsinki Declaration of 1975, as revised in 1983. Within one week of dementia testing, CSF was obtained by lumbar puncture. To control for possible influences of a ventriculo-lumbar gradient, lumbar punctures were done between 7.30 and 8 a. m. before breakfast while patients were still lying flat. CSF samples were frozen on dry ice immediately upon withdrawal at the bedside in 0.5 ml aliquots and stored at - 85 °C until biochemical analysis.

CSF levels of NGF were measured by an ELISA as described recently (Weskamp et al., J. Neurochem. 48, 1779 - 1786, 1987). Black 96-well microplates (Nunc) were coated with monoclonal anti-β (2.5 S, 7S) NGF antibodies (Ab) (clone 27/21, Boehringer Mannheim) diluted in carbonate buffer pH 9.2 over night at 4 °C. 120 µl of CSF and standard solutions were added and incubated for 20 hours at 4 °C. Plates were washed and incubated with anti-β (2.5 S, 7S) NGF-β-galactosidase conjugate for 2 ½ hours at room temperature (RT). Following an additional washing step, the fluorogenic substrate 4-methylumbelliferyl-β-D-galactopyranoside was added and plates were incubated at 4 °C over night. The reaction was stopped after 1 h at RT and the flurorescent product was measured in the microtiter wells using a fluorometer (Labsystems Fluoroskan Ascent FL) (excitation wavelength: 355 nm; emission wavelength: 460 nm). The detection limit was 1.5 pg/ml; the cross-reactivity with other neurotrophins at 10 ng/ml was < 2 %.

ApoE genotyping was performed using INNO-LiPA ApoE, Innogenetics, Belgium. ApoE phenotyping was performed according to McDowell et al. (Clin. Chem. 35(10), 2070 - 2073, 1989). The use of the ApoE phenotype synonymous with the ApoE genotype in the statistical analyses seemed to be appropriate, since ApoE genotyping compared with protein phenotyping showed conflicting results in less than 2 % (Hansen et al., Clin. Chim. Acta, 224(2), 131 - 137, 1994).

Statistical analyses of data were performed using the Mann-Whitney U test for group comparisons. Correlation analyses were performed by multiple regression using CSF levels of neurotrophins as well as ApoE genotype (or phenotype, respectively), age, duration of the disease in AD, MMS, NOSGER and MADRS scores. Regression analysis was complemented with analysis of variance (ANOVA) by using SPSS for Windows (version 8.0). Statistical significance was assumed at p < 0.05. Bonferroni correction for multiple testing was applied.

## Claims

1. A method for diagnosing or prognosing Alzheimer's disease in a subject, or determining whether a subject is at increased risk of developing Alzheimer's disease, comprising:
determining a level of nerve growth factor and/or of a transcription product of a gene coding for nerve growth factor in a sample from said subject;
and comparing said level to a reference value representing a known disease or health status,
thereby diagnosing or prognosing said Alzheimer's disease in said subject, or determining whether said subject is at increased risk of developing Alzheimer's disease.

2. The method according to claim 1, wherein said sample is a body fluid, preferably cerebrospinal fluid.

3. The method according to claim 2, wherein an increase of said level of nerve growth factor in said cerebrospinal fluid from said subject relative to said reference value representing a known health status indicates a diagnosis, or prognosis, or increased risk of said Alzheimer's disease in said subject.

4. A method of monitoring progression of Alzheimer's disease in a subject, comprising:
determining a level of nerve growth factor and/or of a transcription product of a gene coding for nerve growth factor in a sample from said subject;
and comparing said level to a reference value representing a known disease or health status, thereby monitoring progression of said Alzheimer's disease in said subject.

5. A method according to claim 4, further comprising comparing a level of nerve growth factor and/or of a transcription product of a gene coding for nerve growth factor in said sample with a level of at least one of said substances in a series of samples taken from said subject over a period of time.

6. A method of evaluating a treatment for Alzheimer's disease, comprising:
determining a level of nerve growth factor and/or of a transcription product of a gene coding for nerve growth factor in a sample from said subject;
and comparing said level to a reference value representing a known disease or health status,
thereby evaluating said treatment for said Alzheimer's disease.

7. A kit for diagnosis, prognosis, or determination of increased risk of developing Alzheimer's disease in a subject, said kit comprising:
(a) at least one reagent which selectively detects nerve growth factor or a transcription product of a gene coding for nerve growth factor; and
(b) instructions for diagnosing, or prognosing Alzheimer's disease, or determining increased risk of developing Alzheimer's disease by
(i) detecting a level of nerve growth factor and/or of a transcription product of a gene coding for nerve growth factor in a sample from said subject; and
(ii) diagnosing, or prognosing, or determining whether said subject is at increased risk of developing Alzheimer's disease,
wherein an increased level of nerve growth factor and/or of a transcription product of a gene coding for nerve growth factor compared to a reference value representing a known health status;
or a level of nerve growth factor and/or of a transcription product of a gene coding for nerve growth factor similar or equal to a reference value representing a known disease status
indicates a diagnosis, or prognosis, or increased risk of developing Alzheimer's disease.

8. A method of treating or preventing Alzheimer's disease in a subject comprising administering to said subject in a therapeutically effective amount an agent or agents which directly or indirectly affect an activity, or level, or both said activity and level, of at least one substance which is selected from the group consisting of a gene coding for nerve growth factor, its non-coding regulatory elements, a transcription product of a gene coding for nerve growth factor, and nerve growth factor.

9. Use of an agent for treating Alzheimer's disease, wherein said agent directly or indirectly affects an activity, or level, or both said activity and level, of at least one substance which is selected from the group consisting of a gene coding for nerve growth factor, its non-coding regulatory elements, a transcription product of a gene coding for nerve growth factor, and nerve growth factor.

10. A method for identifying an agent that directly or indirectly affects an activity, or level, or both said activity and level, of at least one substance which is selected from the group consisting of a gene coding for nerve growth factor, its non-coding regulatory elements, a transcription product of a gene coding for nerve growth factor, and nerve growth factor, comprising the steps of:
(a) providing a sample containing at least one substance which is selected from the group consisting of a gene coding for nerve growth factor, its non-coding regulatory elements, a transcription product of a gene coding for nerve growth factor, and nerve growth factor;
(b) contacting said sample with at least one agent;
(c) comparing an activity, or level, or both said activity and level, of at least one of said substances before and after said contacting.
